(19) Europäisches Patentamt
European Patent Office
Office européen des brevets

(11) Publication number: **0 123 832**
**B1**

(12) **EUROPEAN PATENT SPECIFICATION**

(45) Date of publication of patent specification: **19.11.87**

(51) Int. Cl.$^4$: **C 07 D 211/22,** C 07 C 57/00

(21) Application number: **84102439.1**

(22) Date of filing: **07.03.84**

(54) **Process for the optical resolution of a mixture of the enantiomers of trans-3-((4-methoxyphenoxy)-methyl)-1-methyl-4-phenylpiperidine.**

(30) Priority: **07.03.83 DK 1115/83**

(43) Date of publication of application:
**07.11.84 Bulletin 84/45**

(45) Publication of the grant of the patent:
**19.11.87 Bulletin 87/47**

(84) Designated Contracting States:
**AT BE CH DE FR GB IT LI LU NL SE**

(56) References cited:
**GB-A-1 422 263**

(73) Proprietor: **A/S FERROSAN**
**Sydmarken 5**
**DK-2860 Soeborg (DK)**

(72) Inventor: **Christensen, Jorgen Anders**
**Olesvej 6**
**DK-2830 Virum (DK)**
Inventor: **Everland, Peer**
**Tordisvej 62**
**DK-2880 Bagsvaerd (DK)**

(74) Representative: **Patentanwälte Leinweber & Zimmermann**
**Rosental 7/II Aufg.**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 123 832 B1

Courier Press, Leamington Spa, England.

**Description**

This invention relates to a novel process for the optical resolution of a mixture of the enantiomers of trans-3-[(4-methoxyphenoxy)-methyl]-1-methyl-4-phenylpiperidine into its optically active pure forms. The (+)-enantiomer of trans-3-[(4-methoxyphenoxy)-methyl]-1-methyl-4-phenylpiperidine is the antidepressive drug femoxetine having the structural formula

(+),trans 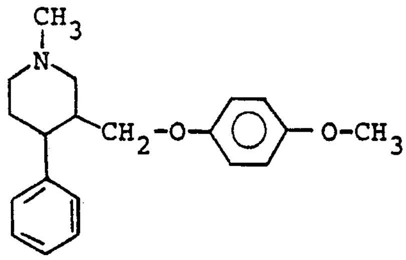

A conventional process for the optical resolution of a racemate into its optically active components comprises reacting the racemate with an optically active resolving agent to form diastereomeric compounds which, because of their different physical properties, can be separated by fractional crystallization from a solvent, in which the solubility of the two compounds is different.

The preliminary step of the conventional process for the optical resolution of a racemate usually comprises the step of mixing equimolar amounts of the racemate and the resolving agent, each dissolved in a suitable solvent or mixing equimolar amounts of a hot solution of the racemate and the resolving agent. Following dissolution of the reactants, the solution is allowed to cool to a temperature at which only one of the diastereomers precipitates. The precipitated diastereomer is then separated from the solution and the precipitate and/or the solution may then be treated by well known procedures to recover one or both optically active compounds.

British patent specification No. 1 422 263 discloses a process for the optical resolution of various racemates of 3-substituted-4-phenylpiperidine compounds including trans-3-[(4-methoxyphenoxy)-methyl]-1-methyl-4-phenylpiperidine by using (−)-dibenzoyltartaric acid as resolving agent.

However, the optical resolution of a mixture of the enantiomers of trans-3-[(4-methoxyphenoxy)-methyl]-1-methyl-4-phenylpiperidine using (−)-dibenzoyltartaric acid as the resolving agent suffers from the defects that the yield obtained is only about 47% of the theoretical value, that the precipitated trans-3-[(4-methoxyphenoxy)-methyl]-1-methyl-4-phenylpiperidine, (−)-dibenzoyltartrate is impure, and that (−)-dibenzoyltartaric acid is relatively costly. The high cost of the (−)-dibenzoyltartaric acid makes it desirable to recover from the precipitated diastereomeric compound, but this has turned out to be difficult. Therefore, the prior art process is of little value for commercial production of e.g. femoxetine.

Surprisingly, it has now been found that an improved optical resolution of a mixture of the enantiomers of trans-3-[(4-methoxyphenoxy)-methyl]-1-methyl-4-phenylpiperidine can be effected with less than the equimolar amount of the resolving agent by using one of the enantiomers of mandelic acid in which the phenyl group is optionally halogen-substituted as resolving agent.

Accordingly, the process of the invention comprises the steps of reacting a mixture of the enantiomers of trans-3-[(4-methoxyphenoxy)-methyl]-1-methyl-4-phenylpiperidine with an optically active resolving agent in an organic solvent to form two diastereomeric compounds, precipitating one of said diastereomeric compounds, separating the precipitated compound from the solution and optionally converting the precipitated and/or the dissolved diastereomeric compound into the free base or a pharmaceutically acceptable salt thereof, and said process is characterized in that the optically active resolving agent used in an enantiomer of mandelic acid and 2-hydroxy-2-phenyl acetic acid, respectively, in which the phenyl group is optionally halogen-substituted.

In a preferred embodiment of the process of the invention the reaction is effected at an elevated temperature, e.g. at a temperature of from 50 to 100°C.

In another preferred embodiment of the invention the precipitation is effected by cooling the solution to a temperature at which only said diastereomeric compound precipitates, e.g. to a temperature below room temperature.

The diastereomeric compounds obtained by using mandelic acid or one of said derivatives thereof as resolving agent exhibit a remarkable difference in solubility and, therefore, it is possible to effect almost a total separation of one of the compounds with one crystallization only.

The organic solvent used as reaction medium is preferably an aromatic solvent including alkylated and halogenated derivatives; an aliphatic ester; an aliphatic alcohol or a mixture of two or more of these solvents. The organic solvent is most preferably toluene, ethyl acetate, methyl isobutyl carbinol or mixtures thereof.

The resolving agent is preferably (+)-mandelic acid or one of said derivatives thereof, most preferably (+)-mandelic acid. Preferred derivatives of mandelic acid are compounds in which the phenyl group is substituted with at least one halogen atom. Particularly preferred are compounds in which the phenyl group comprises a halogen substituted in the 4-position.

When using (+)-mandelic acid as the resolving agent and toluene as the solvent, the reaction is preferably effected at a temperature of 50—75°C and the precipitation is preferably carried out by cooling the solution to a temperature of between 0 and 30°C.

The toluene is preferably used in an amount of 0.3—0.4 l per mole of racemic trans-3-[(4-methoxyphenoxy)-methyl]-1-methyl-4-phenylpiperidine.

The amount of (+)-mandelic acid is preferably from 0.5 to 0.6 per mole of trans-3-[(4-methoxyphenoxy)-methyl]-1-methyl-4-phenylpiperidine. Despite the small amount of the resolving agent used, (+)-trans-3-[(4-methoxyphenoxy)-methyl]-1-methyl-4-phenylpiperidine,(+)-mandelate can be obtained in a yield of approximately 75% of the theoretical value and said mandelate is of a very high purity.

When used for the recovery of (+)-trans-3-[(4-methoxyphenoxy)-methyl]-1-methyl-4-phenylpiperidine in the form of the (+)-mandelate, the process of the invention not only presents the advantage that less than equimolar amounts of resolving agent is required but also the additional advantage that (+)-mandelic acid as well as racemic trans-3-[(4-methoxyphenoxy)-methyl]-1-methyl-4-phenylpiperidine left in the solution can easily be recovered and recycled, thus only leaving the (−)-trans-3-[(4-methoxyphenoxy)-methyl]-1-methyl-4-phenylpiperidine as waste product.

The following examples illustrate the invention:

Example 1

116.4 kg (373.7 mole) of racemic trans-3-[(4-methoxyphenoxy)-methyl]-1-methyl-4-phenylpiperidine were dissolved in 100 l of toluene. The solution was stirred while the temperature was raised to 60°C. 31.2 kg (205.5 mole) of (+)-mandelic acid were added and the mixture was stirred at 60°C until it became clear. The solution was allowed to cool to room temperature. The temperature of the mixture was then reduced to 10°C while stirring during the next 15 hours. The mixture was then centrifuged, and the precipitate was washed with toluene and iso-propanol. The yield was 64.8 kg (75% of the theoretical yield) of (+)-trans-3-[(4-methoxyphenoxy)-methyl]-1-methyl-4-phenylpiperidine,(+)-mandelate, m.p. 126—30°C, $[\alpha]_D^{20}$ + 90.2° (C = 2 in 96% ethanol). Recrystallization of said mandelate salt did not change the value of $[\alpha]_D^{20}$.

50 kg of (+)-trans-3-[(4-methoxyphenoxy)-methyl]-1-methyl-4-phenylpiperidine,(+)-mandelate, was mixed with 27.5 l of distilled water, 67.5 l of toluene and 6.1 l of 50% w/w NaOH to form an aqueous and a toluene phase. The toluene phase was separated and stirred with 3.3 kg of potassium carbonate and filtered. 10 l of concentrated hydrochloric acid and 45 l of isopropanol were mixed with the toluene phase and the mixture was evaporated. The residue was recrystallized from iso-propanol to give 33.8 kg (90%) of (+)-trans-3-[(4-methoxyphenoxy)-methyl]-1-methyl-4-phenylpiperidine as the hydrochloride. M.p. 189—90°C, $[\alpha]_D^{20}$ = 75.3° (C = 5 in $H_2O$).

The toluene solution obtained by the centrifugation of the cooled reaction mixture was mixed with 13 l of 9 M aqueous NaOH and 14 l of water. The mixture was stirred and the aqeuous phase thus obtained was mixed with the above mentioned aqueous phase and heated to 40°C. 23 l of concentrated hydrochloric acid were added to adjust the pH value of about 1.5 and the mixture was cooled and stirred. When the temperature had dropped to about 10°C, the precipitated formed was separated and washed once with 10 l of water giving 26.5 kg of (+)-mandelic acid (85% of theoretical yield). M.p. 130—3°C. Thus, most of the resolving agent was recovered.

Examples 2—4

In a similar manner but with different solvents and mandelic acid and the following derivatives thereof in an amount of 55% on molar basis of racemic trans-3-[(4-methoxyphenoxy)-methyl]-1-methyl-4-phenylpiperidine the procedure of example 1 was repeated. The results obtained will appear from the following table which shows the overall yield of the salt of (+)-trans-3-[(4-methoxyphenoxy)-methyl]-1-methyl-4-phenylpiperidine (femoxetine) and (+)-mandelic acid and the following derivatives thereof.

| Ex. | Solvent | Resolving agent | Overall yield of the salt of femoxetine and (+)-mandelic acid or a derivative thereof (%) |
|---|---|---|---|
| 2 | toluene | (+)-2-hydroxy-2-(4-fluorophenyl)-acetic acid | 74.0 |
| 3 | toluene | (+)-2-hydroxy-2-(4-chlorophenyl)-acetic acid | 66.5 |
| 4 | ethyl acetate | (+)-2-hydroxy-2-phenyl-acetic acid | 67.5 |

These results clearly show that the present invention provides an improved process for the production of femoxetine in that the yield of the salt of femoxetine and (+)-mandelic acid or one of said derivatives is 65—75% of the theoretical value compared to 47% obtained by the known methods. Furthermore, said salt of femoxetine and (+)-mandelic acid or one of said derivatives is of very high purity.

Example 5

The procedure of example 1 was repeated except that the reactants were dissolved in methyl isobutyl carbinol. The yield of (+)-trans-3-[(4-methoxyphenoxy)-methyl]-1-methyl-4-phenylpiperidine,(+)-mandelate was 59% of the theoretical yield.

Example 6

15.0 g (48.2 mmoles) of racemic trans-3-[(4-methoxyphenoxy)-methyl]-1-methyl-4-phenylpiperidine were dissolved in 11.5 ml of toluene. The solution was stirred while the temperature was raised to 60°C. 66.5 g (43.1 mmoles) of (+)-mandelic acid were added and the mixture was stirred at 60°C until it was clear. The solution was allowed to cool to room temperature. The temperature of the mixture was then reduced to 10°C and the mixture was left at this temperature for the next 15 hours. The mixture was then filtered and the precipitate was washed with toluene and isopropanol. The yield was 6.1 g (55% of the theoretical yield) of (+)-trans-3-[(4-methoxyphenoxy)-methyl]-1-methyl-4-phenylpiperidine,(+)-mandelate, m.p. 121°C, $[\alpha]_D^{20}$ + 81.6° (C = 2 in 96% ethanol).

It is surprising to note that the use of a substantially equimolar amount of (+)-mandelic acid as resolving agent gives a yield which is lower than the yield obtained by using (+)-mandelic acid in an sub-equimolar ratio.

Comparison Example

20 g (64 mmoles) of racemic trans-3-[(4-methoxyphenoxy)-methyl]-1-methyl-4-phenylpiperidine and 22 g (62 mmoles) of (−)-dibenzoyl tartaric acid were dissolved in 200 ml of ethanol. The mixture was evaporated under vacuum. The residue was recrystallized from 50 ml of methanol to yield 16.2 g of product. M.p. 84.1—84.6°C, $[\alpha]_D^{20}$ 0.38° (C = 5 in 99% ethanol).

100 g of the product thus obtained were mixed with 20 ml of 4M NaOH, 40 ml of water and 100 ml of ether. The organic phase was dried with potassium carbonate, filtered and evaporated under vacuum to give 4.2 g of product. The product was dissolved in 75 ml of isopropanol. 1 ml of concentrated hydrochloric acid was added and the mixture was evaporated to give 4.7 g of product. M.p. 182.7—184.2°C. 1.9 g of this product was recrystallized from 6 ml of isopropanol to give 1.4 g of (+)-trans-3-[(4-methoxyphenoxy)-methyl]-1-methyl-4-phenylpiperidine hydrochloride. M.p. 189.4°C, $[\alpha]_D^{20}$ 75.6° (C = 5 in $H_2O$). The overall yield of the hydrochloride was 47% of theoretical yield.

**Claims**

1. A process for the optical resolution of a mixture of enantiomers of trans-3-[(4-methoxyphenoxy)-methyl]-1-methyl-4-phenylpiperidine comprising the steps of reacting the mixture with an optically active resolving agent in an organic solvent to form two diasteriomeric compounds, precipitating one of said diasteriomeric compounds, separating the precipitated compound from the solution and optionally converting the precipitated and/or the dissolved diasteriomeric compound into the free base or a pharmaceutically acceptable salt thereof, characterized in that the optically active resolving agent used is an enantiomer selected from a group consisting of mandelic acid in which the phenyl group is optionally halogen-substituted.

2. A process according to claim 1, characterized in using (+)-mandelic acid as resolving agent.

3. A process according to claim 1, characterized in using mandelic acid in which the phenyl group comprises a halogen substituent in the 4-position.

4. A process according to claim 1, characterized in that the reaction is effected at an elevated temperature.

5. A process according to claim 1, characterized in that the precipitation is effected by cooling the solution to a temperature at which only said diastereomeric compound precipitates.

6. A process according to claim 1, characterized in that the organic solvent is an aromatic solvent, an aliphatic ester, an aliphatic alcohol or a mixture of two or more of these solvents.

7. A process according to claim 5, characterized in that the resolving agent is (+)-mandelic acid and that the precipitation is carried out by cooling the solution to a temperature between 0 and 30°C.

8. A process according to claim 1, characterized in that the reaction is effected in toluene heated to a temperature of between 50 and 75°C.

9. A process according to claim 8, characterized in using from 0.3 to 0.4 litres of toluene per mole of the mixture of enantiomers.

10. A process according to claim 1, characterized in that the optically active resolving agent is used in a molar amount which is less than the molar amount of the mixture of the enantiomers.

11. A process according to claim 10, characterized in reacting from 0.5 to 0.6 moles of (+)-mandelic acid with each mole of the mixture enantiomers.

**Patentansprüche**

1. Verfahren zur optischen Trennung einer Enantiomer -Mischung von Trans-3-((4-methoxyphenoxy)-methyl)-1-methyl-4-phenylpiperidin, umfassend die Stufen Umsetzen der Mischung mit einem optisch

aktiven Trennmittel in einem organischen Lösungsmittel zur Bildung von zwei diasteriomeren Verbindungen, Ausfällen einer der genannten diasteriomeren Verbindungen, Abtrennen der ausgefällten Verbindung von der Lösung und gegebenenfalls Umwandeln der ausgefällten und/oder der gelösten diasteriomeren Verbindung in die freie Base oder ein pharmazeutisch verträgliches Salz davon, dadurch gekennzeichnet, daß das benutzte optisch aktive Trennmittel ein Enantiomer ist, ausgewählt aus einer Gruppe bestehend aus Mandelsäure, bei der die Phenylgruppe gegebenenfalls halogensubstituiert ist.

2. Verfahren nach Anspruch 1, gekennzeichnet, durch die Verwendung von (+)-Mandelsäure als Trennmittel.

3. Verfahren nach Anspruch 1, gekennzeichnet durch die Verwendung einer Mandelsäure, bei der die Phenylgruppe in 4-Position einen Halogensubstituenten aufweist.

4. Verfahren nach Anspruch 1, gekennzeichnet, daß die Umsetzung bei einer erhöhten Temperatur ausgeführt wird.

5. Verfahren nach Anspruch 1, gekennzeichnet daß die Ausfällung durch Abkühlen bis zu einer Temperatur bewirkt wird, bei der nur die gennante diasteriomere Verbindung ausfällt.

6. Verfahren nach Anspruch 1, gekennzeichnet, daß das organische Lösungsmittel ein aromatisches Lösungsmittel, ein aliphatischer Ester, ein aliphatischer Alkohol oder eine Mischung von 2 oder mehr dieser Lösungsmittel ist.

7. Verfahren nach Anspruch 5, gekennzeichnet, daß das Trennmittel (+)-Mandelsäure ist und die Ausfällung durch Abkühlen der Lösung auf eine Temperatur zwischen 0 und 30°C ausgeführt wird.

8. Verfahren nach Anspruch 1, gekennzeichnet, daß die Umsetzung in Toluol ausgeführt wird, das auf eine Temperatur zwischen 50 und 75°C erhitzt ist.

9. Verfahren nach Anspruch 8, gekennzeichnet durch die Verwendung von 0,3 bis 0,4 l Toluol/Mol der Mischung von Enantiomeren.

10. Verfahren nach Anspruch 1, gekennzeichnet, daß das optisch aktive Trennmittel in einer molaren Menge eingesetzt wird, die geringer ist als die molare Menge der Mischung der Enantiomeren.

11. Verfahren nach Anspruch 10, gekennzeichnet durch die Umsetzung von 0,5 bis 0,6 Mol der (+)-Mandelsäure mit jedem Mol der Mischung der Enantiomeren.

**Revendications**

1. Procédé de résolution optique d'un mélange d'énantiomères de trans-3-[(-méthoxyphénoxy)-méthyl]-1-méthyl-4-phénylpipéridine, comprenant les étapes de réaction du mélange avec un agent optiquement actif d résolution dans un solvant organique pour former deux composés diastéréo-isomériques, précipitation de l'un de ces composés diastéréo-isomériques, séparation du composé précipité de la solution et, à titre facultatif, transformation du composé diastéréo-isomérique précipité et/ou du composé diastéréo-isomérique dissous en la base libre ou en l'un de ses sels pharmaceutiquement acceptables, caractérisé en ce que l'agent optiquement actif de résolution utilisé est un énantiomère choisi dans le groupe constitué de l'acide mandélique dont le group phényle est facultativement substitué par un halogène.

2. Procédé suivant la revendication 1, caractérisé par l'utilisation de l'acide (+)-mandélique comme agent de résolution.

3. Procédé suivant la revendication 1, caractérisé par l'utilisation d'acide mandélique dont le groupe phényle comprend un substituant halogéno en position 4.

4. Procédé suivant la revendication 1, caractérisé en ce que la réaction est conduite à une température élevée.

5. Procédé suivant la revendication 1, caractérisé en ce que la précipitation est conduite par refroidissement de la solution à une température à laquelle seul le composé diastéréo-isomérique précipite.

6. Procédé suivant la revendication 1, caractérisé en ce que le solvent organique est un solvant aromatique, un ester aliphatique, un alcohol aliphatique ou un mélange de deux ou plus de deux ces solvants.

7. Procédé suivant la revendication 5, caractérisé en ce que l'agent de résolution est l'acide (+)-mandélique et en ce que la précipitation est conduite par refroidissement de la solution à une température comprise entre 0 et 30°C.

8. Procédé suivant la revendication 1, caractérisé en ce que la réaction est conduite dans du toluène chauffé à une température comprise entre 50 et 75°C.

9. Procédé suivant la revendication 8, caractérisé par l'utilisation de 0,3 à 0,4 litre de toluène par mole due mélange d'énantiomères.

10. Procédé suivant la revendication 1, caractérisé en ce que l'agent optiquement actif de résolution est utilisé en une quantité molaire qui est inférieure à la quantité molaire de mélange des énantiomères.

11. Procédé suivant la revendication 10, caractérisé par la réaction de 0,5 à 0,6 mole d'acide (+)-mandélique avec chaque mole du mélange d'énantiomères.